Europäisches Patentamt

European Patent Office

Office européen des brevets

(11) Publication number: **0 249 932**
A2

# EUROPEAN PATENT APPLICATION

(21) Application number: 87108626.0

(22) Date of filing: **16.06.87**

(51) Int. Cl.⁴: **C 07 K 3/28**, C 07 K 17/02
// A61K37/02

(30) Priority: **17.06.86 JP 139142/86**

(43) Date of publication of application: **23.12.87**
**Bulletin 87/52**

(84) Designated Contracting States: **BE CH DE ES FR GB LI NL SE**

(71) Applicant: **THE GREEN CROSS CORPORATION, 15-1, Imabashi-1-chome Higashi-ku Osaka-shi, Osaka 541 (JP)**

(72) Inventor: **Toshiharu, Motokubota, 5-3-10, Nanbeidai, Takatsuki-shi Osaka (JP)**
Inventor: **Hiroshi, Morise, 1-50-5, Higashiyama, Hirakata-shi Osaka (JP)**

(74) Representative: **Hansen, Bernd, Dr.rer.nat. et al, Hoffmann, Eitle & Partner Patentanwälte Arabellastrasse 4, D-8000 München 81 (DE)**

(54) Automatic process for purifying physiologically active substance, apparatus for the same, and carrier with proteinaceous ligand fixed thereto to be used for the same.

(57) An automatic process for isolating and purifying physiologically active substances contained in large quantities of sample solutions, which comprises the following three steps: (1) filtration (a step for removing microbial cells and other insoluble matters), (2) ultrafiltration (a step for removing impurity having a molecular weight lower than that of the active substance, and concentrating the sample solution), ans (3) affinity chromatography using a carrier with a substance having specific affinity to the active substance fixed thereto (a step for isolating pure product by selective adsorption and elution), an apparatus for use in the same and a carrier employed in the same.

EP 0 249 932 A2

AUTOMATIC PROCESS FOR PURIFYING PHYSIOLOGICALLY
ACTIVE SUBSTANCE, APPARATUS FOR THE SAME,
AND CARRIER WITH PROTEINACEOUS LIGAND
FIXED THERETO TO BE USED FOR THE SAME

## FIELD OF THE INVENTION

This invention relates to an automatic process for purifying physiologically active substances, to an apparatus for the same, and to a carrier with a proteinaceous ligand fixed thereto to be used for the same.

## BACKGROUND OF THE INVENTION

Various techniques are known for isolating and purifying small amounts of physiologically active substances from large quantities of sample solutions (for example, culture solutions of microbial and other cells, extracts therefrom, untreated urine and sera, etc.).

Typical    examples of these techniques include   filtration, ultrafiltration, centrifugal separation, hydrophobic chromatography, ion-exchange chromatography, affinity chromatography, gel filtration, fractionation using polyethylene glycol or ammonium sulfate  and salting out.

It is generally possible to obtain physiologically active substances of sufficiently high purity by combinations of these purification techniques appropriate to the particular substances to be isolated.

However, there is a problem in that much time and labor are required during transferring from one step to the next (for example, for transferring treated solutions from one apparatus to another). Thus, when more purification steps are combined to achieve higher product purity, the operations become more cumbersome and takes a longer time for completion. This disadvantage is particularly true when a plurality of physiologically active substances in a sample solution are to be isolated and purified. In addition, in manual operations it is difficult to maintain germ-free conditions throughout the entire course of purification.

## SUMMARY OF THE INVENTION

The problems associated with conventional method can be avoided by the automatic process of this invention comprising the following steps:

(1) filtering a sample solution containing a physiologically active substance (or substances) through at least one filter of a pore size in the range of from 0.22 to 500 μm to give a clarified solution containing the physiologically active substance,

(2) subjecting, if desired, the clarified solution thus obtained to ultrafiltration, thereby filtering off impurity substances having a molecular weight lower than that of the physiologically active substance so as to produce a concentrated solution of the physiologically active substance,

(3) subjecting the clarified or concentrated solution containing the physiologically active substance to affinity chromatography, to thereby obtain a purified solution containing the active substances.

The problems associated with conventional methods can also be avoided by the use of an apparatus comprising:

(1) a tank for storing a solution containing a physiologically active substance to be purified,

(2) a filter of a pore size in the range of from 0.22 to 500 µm for filtering the solution stored in tank (1),

(3) a tank for storing the filtrate obtained from filter (2) and ultrafilter (4),

(4) an ultrafilter, if desired, for filtering the solution stored in tank (3) to filter off impurity substances having a molecular weight lower than that of the physiologically active substance to be purified, to thereby obtain a concentrated solution of the active substance,

(5) a separator through which the concentrated solution obtained above is passed and which contains a carrier with a substance having specific affinity to the physiologically active substance fixed thereto,

(6) a tank for storing a washing solution, an equilibrating solution, or both for use with separator (5),

(7) a tank for an eluent for eluting the physiologically active substance adsorbed on separator (5),

(8) a tank for storing the fractions eluted from separator (5) above and containing the physiologically active substance,

(9) pipes for transferring the solution from tank (1) to filter (2) to tank (3) and from tank (3) to ultrafilter (4),

(10) a pipe for transferring the solution withdrawn from ultrafilter (4) to tank (3),

(11) pipes for transferring the solutions in tanks (3), (6) and (7) to separator (5),

(12) a pipe for transferring the solution withdrawn from separator (5) to tanks (8) and other units, e.g., a fraction collector,

(13) delivery pumps provided in pipes (9), (10) and (11),

(14)  valves provided in pipes (9), (10) and (11),

(15)  liquid level sensors provided in tanks (1) and (3),

(16)  a pressure sensor provided in the pipe between tank (3) and ultrafilter (4) and in pipe (10), and

(17)  a computer which is electrically connected to sensors (15) and (16) at its input and to valve (14) at its output.

## BRIEF DESCRIPTION OF THE DRAWINGS

Figure 1 is a schematic illustration of an automatic purification apparatus when the sample solution applied is a culture solution of animal cells, blood or urine.

Figure 2 is a schematic illustration of an automatic purification apparatus when the sample solution applied is a culture solution of Escherichia coli, yeast or Bacillus subtilis carrying a recombinant gene, or an extract therefrom.

Figure 3 is a flowchart for computor control of an automatic purification apparatus.

Connection between the computer and valves is omitted in these figures.

## DETAILED DESCRIPTION OF THE INVENTION

The process of this invention is described in detail below.

(1)  Pretreatment

This is a step before the starting material is sent to the filtration step.

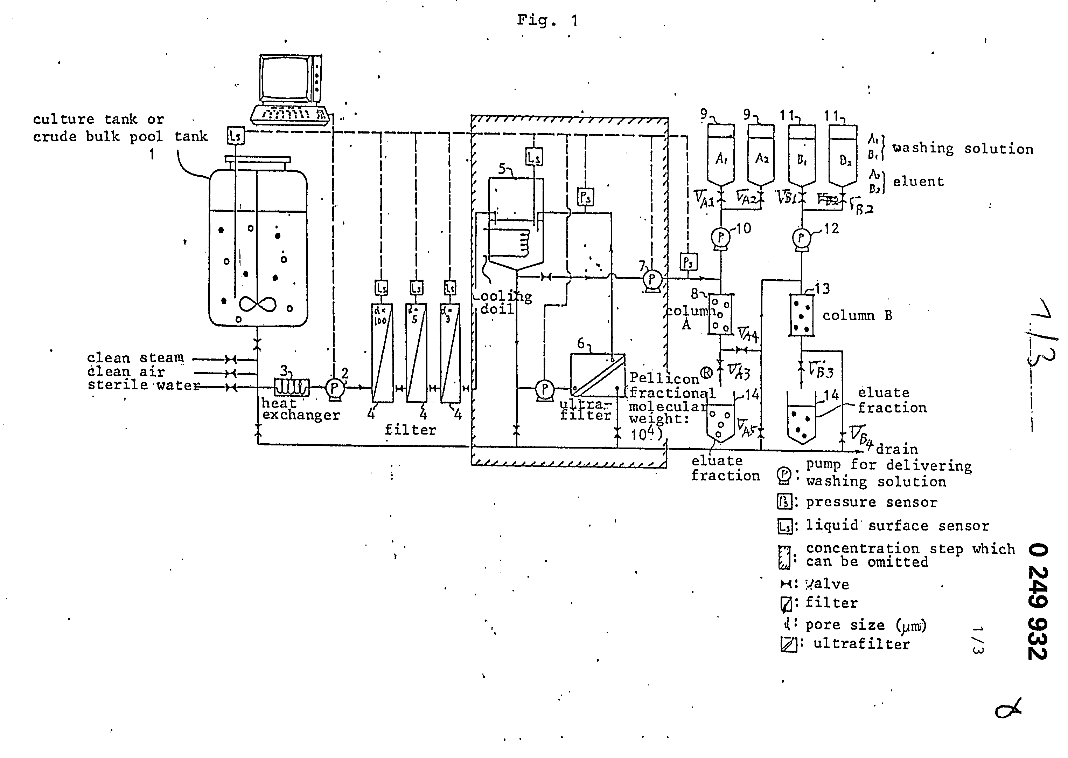

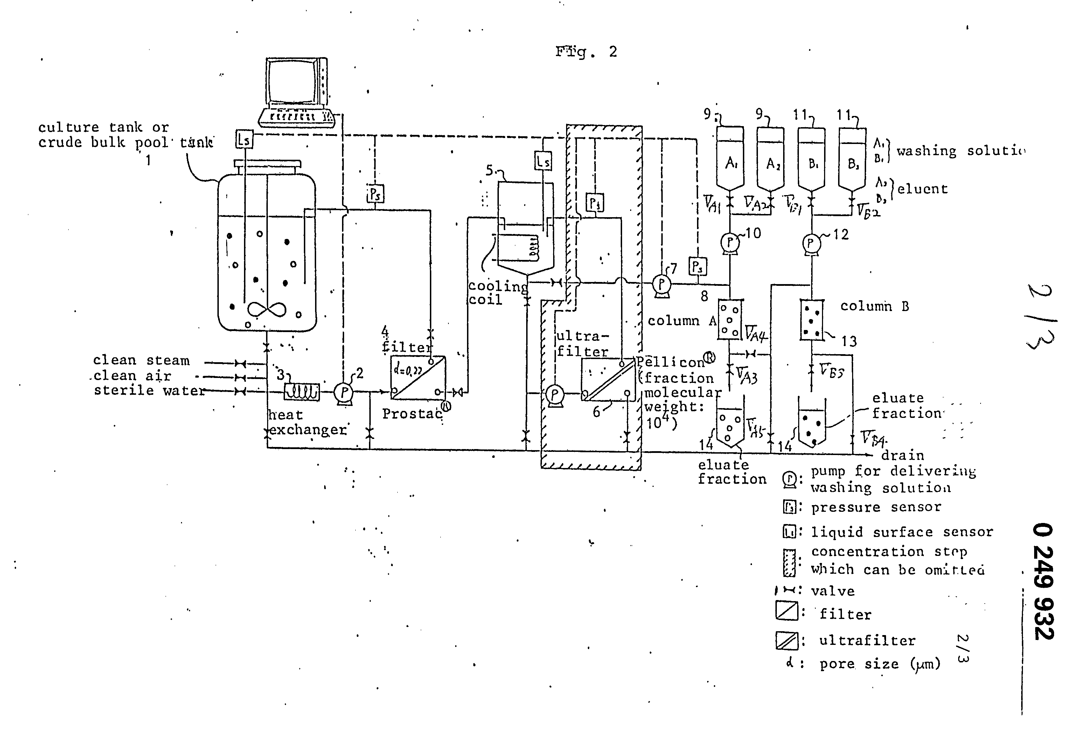

The units used in this step include tank 1 for storing a solution containing a physiologically active substance (e.g., fermentation tank or pool tank for crude, untreated sample solutions to be purified), delivery pump 2 and cooler 3. These units are connected as shown in Figure 1 or 2 in actual practice.

Typical examples of the starting material used in this invention (solutions containing physiologically active substances) are culture solutions of various types of cells, including leucocytes, lymphoblasts, hybridomas and many other cultured cells. Culture solutions of _Escherichia coli_, _Bacillus subtilis_, yeast and animal cells incorporating genes capable of producing physiologically active substances, extracts therefrom, urine, sera and plasma are also included. There is no specific limitation upon the purification degree of these starting materials, but the effectiveness of this invention is more marked when starting materials of lower purity are applied.

There is also no specific limitation upon the type of physiologically active substance to be isolated and purified. Illustrative examples include various types of interferons (IFN-α, IFN-β, IFN-γ), urokinase (UK), prourokinase (pro UK), kallikrein (KL), lysozyme, trypsin inhibitor, human granulocyte-differentiation-inducing glycoprotein (HGIGP), various types of interleukins (type-1, -2, -3), colony-formation-stimulating factor (CSF), tissue-plasminogen-activating factor (TPA), B-cell growth factor (BCGF), tumor necrosis factor (TNF), epidermal growth factor (EGF), lymphotoxin (LT), various types of hormones, various types of monoclonal antibodies, erythropoietin (EPO), antithrombin III (AT-III), trans-ferrin, plasminogen, plasminogen activator, $\alpha$-fetoprotein (AFP), surface antigen for hepatitis type-B virus ($HB_sA_g$), $HG_sA_g$ containing HB virus Pre S region, and combinations thereof.

In this pretreatment step, the solution in storage tank 1 is sent to the next step by the action of derivery pump 2 preferably at a rate of 1 to 100 ℓ/hr and more pre-ferably 20 to 60 ℓ/hr. It is preferable that the solution be maintained by cooler 3 at a temperature in the range of from 0 to 10°C, preferably from 4 to 10°C, during delivery.

(2) Filtration

The object of this step is to clarify the solution to be treated by removing solid matters suspending therein (e.g., microbial and other cells, and fragments thereof).

The units used in this step include a plurality of filters 4 of pore size ranging from 0.22 to 500 μm, and tank 5 for storing the filtrate. These are connected as shown in Figure 1 or 2 in actual practice.

It is preferable to use several filters 4 of different pore sizes in combination. The pore size and combination of filters 4 should be selected depending on the conditions of the particular solution to be treated.

For example, filters of 3 μm, 0.45 μm and 0.22 μm pore sizes are used for medium from cloned cells, and those of 100 μm, 5 μm, 3 μm, 0.45 μm and 0.22 μm pores sizes are employed in that order for medium from  leucocytes to remove  insoluble proteins and lumps of cells.  For the medium for microbial cells obtained by the gene recombination technique or extracted suspensions thereof, a combination of filters of 0.45 μm and 0.22 μm pore sizes is preferable.  Particularly, in the case with recombinant cells of Escherichia coli, Bacillus subtilis and yeast, high-pressure, high-speed circulating filtration of the ultrafiltration type (Prostack[®]; pore size: 0.22 μm; Millipore Inc.) is especially effective.

The clarified solution sent from  pretreatment step (1) is allowed to pass through a series of filters 4 of 0.2 to 500 μm pore sizes, and the filtrate thus obtained is stored in    storage tank 5 preferably at 0 to 10°C (most preferably at about 4°C).

(3)  Ultrafiltration

The object of this step is to concentrate the filtrate obtained in step (2).  The main unit in this step is an ultrafilter 6 capable of filtering off substances having a molecular weight lower than that of the physiologically active substance to be purified and giving a concentrated solution of that active substance. The solution stored in tank 5 of step (2) is pumped into ultrafilter 6 generally at a rate of 10 to 1,000 ℓ/hr and preferably 50 to 500 ℓ/hr at a temperature of generally from 0 to 10°C and preferably 4 to 10°C, the filtrate thus obtained is circulated back to storage tank 5, and this operation is preferably repeated until 10 to 1,000 liters of solution is concentrated to 0.1 to 5 liters. Concentration of this step may be omitted when the volume of sample solution is about 100 liters or less. The time required for this concentration step is 0.5 to 10 hours.

(4) Affinity Chromatography

The object of this step is to isolate and purify the physiologically active substance by using a column packed with a carrier to which a substance having specific affinity to that active substance has been fixed.

The units used in this step include pump 7 for transferring the solution in storage tank 5 in step (3), to column 8 packed with a carrier to which a substance having specific affinity to that physiologically active substance has been fixed, tank 9 for the storing washing solution and/or equilibrating solution, a second pump 10 for transferring the washing solution to the column, a second storage tank 11 for the eluent for eluting the physiologically active substance adsorbed on the carrier, a third pump 12 for transferring the eluent to the column 13, and a third tank 14 for storing the fractions containing the physiologically active substance eluted. These units are connected as shown in Figure 1 or 2 in actual practice. The solution in storage tank 5 is fed to column 8 at a rate of generally 10 to 300 m$\ell$/cm$^2$/hr and preferably 50 to 200 m$\ell$/cm$^2$/hr at a temperature of generally 0 to 10°C and preferably 4 to 10°C. On the other hand, the washing solution in storage tank 9 or 11 is fed to column 8 or 13 at a rate of generally 100 to 2,000 m$\ell$/cm$^2$/hr and preferably 500 to 1,500 m$\ell$/cm$^2$/hr at a temperature of generally 0 to 10°C and preferably 4 to 10°C. The element in storage tank 9 or 11 is fed to column 8 or 13 at a rate of generally 10 to 300 m$\ell$/cm$^2$/hr and preferably 50 to 200 m$\ell$/cm$^2$/hr at a temperature of generally 0 to 10°C and preferably 4 to 10°C.

Various substances having specific affinity to physiologically active substances are known. These include antibodies (both polyclonal and monoclonal) against various antigens, antigens against various antibodies, enzymes against various substrates, substrates against various enzymes, benzamidine, concanavalin A (ConA), aprotinin, heparin, and metal ions ($Cu^{2+}$, $Zn^{2+}$, $Ni^{2+}$, etc.). Many combinations of physiologically active substances and substances having specific affinity there- to are known. Typical examples are listed in Table 1.

As the insoluble carrier, amino acid copolymers, cellulose, agarose (e.g., Sepharose®), dextran (e.g., Sephadex®), polyacrylamide, chitosan beads (e.g., Chitopearl®) and porous glass (e.g., silica gel and controlled pore glass (CPG)) may be employed.

For the treatment of large quantities of sample solutions, it is important that the carrier used have sufficiently high pressure resistance, otherwise it tends to be deformed or broken during service which clogs the column, thus significantly lowering the flow rate or even stopping the flow in extreme cases. Preferred carriers are Formyl-cellulofine (registered trademark to Seikagaku Kogyo Co., Ltd.), chitosan beads, and porous glass (silica gel, CPG, etc.), each being available in various grain and pore sizes to match the properties of the solutions to be treated. Of these, porous glass is inexpensive and is rather readily available in various grain and pore sizes. Ligands can be fixed to porous glass by any known technique, for example, by the method described in Biochemical Journal, No. 117, pp. 257 to 261 (1970); porous glass is first allowed to react with a silane coupling agent (e.g., γ-glycidoxypropyltrimethoxysilane, γ-aminopropyltriethoxysilane, γ-mercaptopropyltrimethoxysilane, γ-chloropropyltrimethoxysilane, γ-cyanopropyltrimethoxysilane, vinyltriethoxy-

silane, and γ-methacryloxypropyltrimethoxysilane) to give activated-glass, followed by fixing a proteinous ligand by the glutaraldehyde method (or by the periodide, carbodiimide and other methods).

Of the various types of porous glass available, silica gel which is granular in shape and has a pore size of 100 Å or larger is particularly suitable for the purpose of this invention (for example, Microbeads Silica Gel; Fiji-Devison).

Similar effects can also be obtained by the use of cartridges which comprise a cellulose membrane shaped in a filter form and containing a proteinous ligand fixed thereto (e.g., ZETAF-FINITY[®]; Cuno Inc. (formerly AMF Inc.)).

The process of this invention also requires an equilibrating solution for equilibrating columns packed with ligand-fixed carriers, an eluent for eluting adsorbed physiologically active substances, and a washing solution for washing columns. The same type of solution may be used for both equilibration and washing.

These solutions will vary depending on the type of physiologically active substance to be purified and the kind of ligand (substance having specific affinity). There are many known combinations for these substances and solutions, typical examples of which are listed in Table 1.

| Ligand | Physiologically Active Substance | Equilibrating Solution (example) | Eluent (example) |
|---|---|---|---|
| Anti-IFN-α antibody | IFN-α | pH 6-8, low ionic strength (0.5 M NaCℓ or less) | pH 2-4 (0.1-0.5 M citric acid) |
| Anti-IFN-β antibody | IFN-β | ditto | ditto |
| Anti-IFN-γ antibody | IFN-γ | ditto | pH 6-8, high ionic strength (1-4 M KSCN, 3-5 M MgCℓ$_2$) |
| Anti-pro-UK antibody | pro UK | ditto | pH 2-4 (0.5 M NaCℓ + 0.2 M Gly·HCℓ (pH 2.5)) |
| Anti-UK antibody | UK | 0.5 M NaCℓ + 0.01 M PB (pH 7) | 0.5 M NaCℓ + 0.1 M Gly·HCℓ (pH 3) |
| Anti-KL antibody | KL | 0.15 M NaCℓ + 0.05 M PB | 0.2 M Na$_2$CO$_3$ |
| Anti-EPO antibody | EPO | 0.15 M NaCℓ + 0.01 M PB | 0.15 M NaCℓ + 0.2 M acetic acid |
| Anti-TNF antibody | TNF | pH 6-8 (0.4 M NaCℓ + 0.1 M PB (pH 7)) | pH 6-8 (3.5 M KSCN + 0.5 M NaCℓ (pH 8)) |
| Anti-CSF antibody | CSF | pH 6-8, low ionic strength (0.5 M NaCℓ or less) | pH 6-8 (1-4 M KSCN + 3-5 M guanidine HCℓ) |
| Anti-LT antibody | LT | ditto | ditto |
| HB$_s$A$_g$ | Anti-HB$_s$A$_g$ antibody | ditto | ditto |

(cont'd)

| Ligand | Physiologically Active Substance | Equilibrating Solution (example) | Eluent (example) |
|---|---|---|---|
| | Trypsin | 0.5 M NaCl + 0.05 M Tris·HCl (pH 8) | 0.5 M NaCl + 0.01 M HCl (pH 2) |
| Benzamidine | KL | 0.4 M NaCl + 0.05 M Tris·HCl (pH 9) | 0.5 M NaCl + 0.1 M Gly·HCl (pH 4) |
| | UK | 0.4 M NaCl + 0.1 M PB (pH 7) | 0.4 M NaCl + 0.1 M acetic acid (pH 4) |
| poly HSA | Pre S-HB A | pH 6-8, low ionic strength (0.5 M NaCl or less) | pH 6-8 (1-4 M KSCN, 3-5 M guanidine HCl) |
| ConA | KL | 1M NaCl | 1M NaCl + 10% Glc |
| Aprotinin | KL | 0.5 M NaCl + 0.05 M PB (pH 8.5) | 1M NaCl + 0.1 M acetic acid (pH 4) |

Note)  PB: phosphate buffer

HSA: human serum albumin

When a plurality of physiologically active substances are to be isolated and purified simultaneously, corresponding affinity columns are serially connected, and tanks for the washing solution, for the eluent and for the eluted active fractions are connected to each column. In the process of this invention, the filtrate from step (1) or the concentrate from step (2) is automatically pumped to this group of affinity columns to allow each of the physiologically active substances contained in the sample solution to be adsorbed on the corresponding column. Each washing solution is allowed to flow to wash the corresponding column, each eluent is then introduced to elute each of the adsorbed physiologically active substances, and the eluate is stored in the tank below each column.

The proteinaceous ligand-fixed carrier used in the process of this invention should preferably be sterilized in order to prevent internal contamination of the line during the purification process and to make the culture medium after the physiologically active substances have been recovered reusable without any treatment, thereby significantly lowering the medium costs.

Any proteinaceous ligands that can be used for affinity chromatography can be used in the process of this invention. Among other things antibody-fixed carriers have excellent purifying effects and are indispensable for the purification of physiologically active substances. The antibodies may be monoclonal or polyclonal. Illustrative examples of the antibodies include antibodies against various types of interferons (IFN-$\alpha$, IFN-$\beta$, IFN-$\gamma$), tissue-plasminogen-activating factor (TPA), various types of interleukins (IL-1, IL-2, IL-3), colony-formation-stimulating factor (CSF), tumor necrosis factor (TNF), epidermal growth factor (EGF), lymphotoxin (LT), B-cell growth factor (BCGF), urokinase (UK), pro-urokinase (pro UK), erythropoietin (EPO), kallikrein (KL), $\alpha$-fetoprotein (AFP), antithrombin III (AT-III), plasminogen, $HB_s$, $HB_e$, $HB_c$, carcinoembryonic antigen (CEA), ferritin and $\beta_2$-macroglobin.

The present invention can also be applied to protein A-carrier which is used for the purification of monoclonal antibodies produced by culturing hybridomas and the like.

Any known carriers for the fixation of proteinaceous ligands may be used for the process of this invention. These include cellulose, dextran, porous glass (silica gel, controlled-pore glass (CPG), etc.), chitosan, agarose, polyacrylamide, ceramics, nylon and amino acid copolymers. Of these, cellulose, porous glass and chitosan are most preferred.

There is no specific limitation upon the shape of carriers; these may be used in the form of granules, fragments, membrane, fiber, tubes or plates.

As examples of commercially available carriers for fixation may be mentioned Sepharose (Pharmacia), Formyl-cellulofine (Seikagaku Kogyo), CPG (Electronucleonics), Microbeads Silica Gel (Fuji-Devison), TRISACRYL (LKB), Chitopearl (Fuji Spinning) and ZETAFFINITY (Cuno).

Fixation of proteinaceous ligands to these carriers may also be effected by any known techniques, for example, by the cyanogen bromide, glutaraldehyde, periodic acid, or silanation method.

The proteinaceous ligand-fixed carrier thus prepared is then sterilized by heating, UV irradiation or other treatments. Of these, thermal treatment is most preferred, which is conducted under conditions to deactivate bacteria and viruses that might be present in the system, for example, at a temperature in the range from 55 to 70°C for 1 to 15 hours. It is preferable to immerse the proteinaceous ligand-fixed carrier in water held at a pH level of 5 to 8.

This thermal treatment is preferably carried out in the presence of a stabilizer to prevent decomposition of the fixed proteinaceous ligand. Suitable stabilizers are sugars (e.g., sucrose, maltose and raffinose), sugar alcohols (e.g., sorbitol), amino acids (e.g., glycine, alanine and arginine), and combinations thereof. Suitable amounts to be added are generally 30 to 90% (w/v) and preferably 30 to 60% (w/v) for sugars and sugar alcohols, and generally 5 to 20% (w/v) and preferably 10 to 20% (w/v) for amino acids.

The sterilized proteinaceous ligand-fixed carriers thus obtained should preferably be stored at about 4°C with an aqueous solvent of low ionic strength (pH: 6 to 8) included therein. A known preservative (e.g., sodium azide) may also be added to the stored carrier, in an amount of about 0.1%, for example.

The proteinaceous ligand-fixed carrier of this invention is usually employed in the form of a cartridge or column.

A series of filters are set at the outlet of a fermentation tank or pool tank (germ-free), and the column or cartridge containing the proteinaceous ligand-fixed carrier is then connected to the end of the filters. Microbial cells, fragments thereof and other insoluble matters are removed while the solution being treated is passed through the filters. The filters should preferably be made of a material that withstands sterilizing treatment (e.g., autoclave and steam sterilization). Such a purification line as described above can be easily assembled, and the culture solution remains germ-free after passing through this system, the culture solution can be reused without any treatment.

(5)  Automatic Control Section

This automatically controls the delivery pumps and valves in steps (1) through (4) described above. The units used in this section include a computer, liquid level sensors and pressure sensors.  The sensors are electrically connected to the input of the computer, while the pumps and valves are connected to its output. Based on  a  built-in program, the computer issues signals to the pumps and valves depending on the information sent from the sensors, thereby automatically controlling the purification process.

More particularly, the liquid level sensor for tank 1 controls pump 2 (when the liquid surface in tank 1 is on or above a predetermined level, i.e., the amount of the liquid in tank 1 is large, pump 2 is actuated  and when it is below the predetermined level, i.e., the amount of the liquid in tank 1 is small, this is detected by the liquid level sensor and signal is sent to pump 2 to stop it).  The liquid sensor for filter 4 controls pump 2 (when the liquid surface in filter 4 is on or below a predetermined level, i.e., the amount of  the liquid in tank 1 is not so large, pump 2 is actuated while the liquid surface is above the predetermined level, i.e., the amount of the liquid is excessively large, this is detected by the liquid level sensor and pump 2 is stopped). The liquid level sensor for tank 5 controls pump 2 (when the liquid surface in Tank 5 is on or below a predetermined level, i.e., the amount of the liquid in tank 5 is not so large, pump 2 is actuated while it is above the predetermined level, i.e., the amount of the liquid is too large, this is detected by the liquid level sensor and pump 2 is stopped). Further, the liquid level sensor for tank 5 controls the valve on the side of filter 6 and the pump for filter 6 (when the liquid surface in tank 5 is on or above a predetermined level, i.e., the amount of the liquid is large, this is detected by the liquid surface sensor and the valve on the side of filter 6 is closed and the pump for filter 6 is actuated while lowering of the liquid surface in tank 5 below a predetermined level, i.e., reduction in the amount of the liquid, is detected by the liquid level sensor and as a result the valve on the side of filter 6 is closed and the pump for filter 6 is stopped). The pressure sensor on the side of tank 5 controls the pump for filter 6 (when the pressure of the liquid after ultrafiltration is on or below a predetermined level, i.e., the pressure caused by the transfer of the liquid is not so high, this is detected by the pressure sensor and the pump is actuated while the pressure sensor detects elevation of the pressure of the liquid above the predetermined level, i.e., the pressure caused by the transfer of the liquid being too high, and send a signal to stop the pump). The liquid level sensor for tank 5 controls pump 7 and the valve on the side of pump 7 (when the liquid surface falls to a predetermined level this is detected by the liquid sensor which sends signals to open the valve on the side of pump 7 and actuate pump 7 while when the liquid level falls to the minimum value, i.e., the amount of the liquid in tank 5 approaches 0 (zero), this is detected by the liquid level sensor, which sends signals to close the valve on the side of pump 7 and stop pump 7). The pressure sensor on the side of pump 7 controls pump 7 (the pump is actuated when the pressure of the liquid fed from tank 5 to column A is not so high while the pressure of the liquid is on or above a predetermined level, i.e., the pressure of the liquid is too high, this is detected by the pressure sensor, which sends a signal to stop the pump).

The automatic purification process and the apparatus for the same of this invention allow purification of physiologically active substances under germ-free conditions in a short period of time, i.e., about from 1 to 24 hours.

- 23 -

The physiologically active substances that can be automatically and continuously isolated and purified by the process and apparatus of this invention include: IFN-α, IFN-γ and TNF from leucocyte culture solutions; UK, KL, lysozyme, trypsin inhibitor, EPO and HGIGP from human urine; various activating factors (e.g., AT-III, plasminogen, celluloplasmin, XIII factor, lysozyme, AFP and UK inhibitor from placenta extracts; and IFN-α, IFN-β, IFN-γ, TNF, CSF, TPA, EGF, LT, EPO, BCGF, UK and pro UK produced by recombinant genes.

The sterile proteinaceous ligand-fixed carriers of this invention are of great use as carrier for affinity chromatography in a closed system (in-line system). Sample solutions (e.g., culture solutions containing valuable substances), after being passed through filters (sterilized as usual with high-pressure, high-temperature steam) for removal of cells, fragments thereof and other insoluble matters, and through the carrier of this invention for adsorption of the valuable substances, still remain germ-free and hence can be reused without any treatment. This contributes to a drastic reduction in production costs of the valuable substances.

The following non-limiting examples will further illustrate the invention.

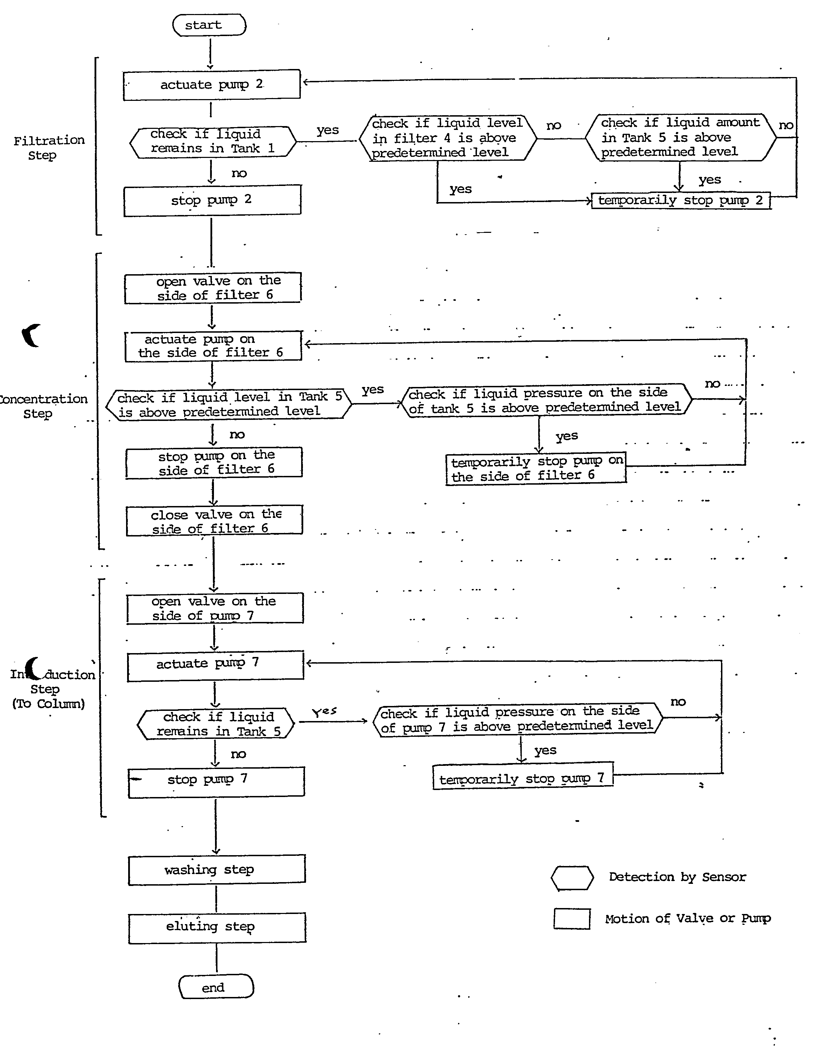

In the examples computor control as shown in Fig. 3 is used.

Motion of valves and pumps used for column are as shown in Table below.

| | | Charging Step | Washing Step | Eluting Step |
|---|---|---|---|---|
| Pump | P7 | actuated | stopped | stopped |
| | P10 | stopped | actuated | actuated |
| | P12 | stopped | actuated | actuated |
| Valve | | | | |
| Column | $V_{A1}$ | closed | open | closed |
| | $V_{A2}$ | closed | closed | open |
| | $V_{A3}$ | closed | closed | open |
| | $V_{A4}$ | open | open | closed |
| | $V_{A5}$ | closed | open | closed |
| Column | $V_{B1}$ | closed | open | closed |
| | $V_{B2}$ | closed | closed | open |
| | $V_{B3}$ | closed | closed | open |
| | $V_{B4}$ | open | open | closed |

The names of the valves are as indicated in Figs. 1 and 2.

## EXAMPLE 1

IFN-α and IFN-β derived from cultured lymphoblasts Production System:

Sendai virus (HVJ) was added to a culture solution of lymphoblasts to induce production of IFN-α and -β. The volume of the culture solution treated was 300 liters.

Purification Procedure:

The apparatus shown in Figure 1 was used, in which one filter of 3.0 μm pore size (Model AB2U030; Pole Inc.) and two columns (A and B) were connected. Column A was packed with 500 mℓ of silica gel (Microbeads Silica Gel, 500 Å; Fuji-Devison) with polyclonal antibody against IFN-α fixed thereto, and column B was packed with 100 mℓ of the same silica gel as above with monoclonal antibody against IFN-γ fixed thereto.

A 0.1 M phosphate buffer containing 0.3 M NaCℓ (pH 7.0) was used as the column equilibrating and washing solutions ($A_1$ and $B_1$) each in an amount ten times the volume of the column.

A 0.1 M citrate buffer (pH 2.0) was used as the column eluent ($A_2$ and $B_2$) each in an amount three times the volume of the column.

The time required for treatment was 4.5 hours.

Result of Purification:

IFN-α and -β were isolated and purified simultaneously using an ultrafilter Pellicon® (casette filter: PTGC) produced by Millipore Inc. as shown in Table 2.

TABLE 2

| Sample | Volume (ℓ) | Total Protein ($A_{280\ nm}$) | Total IFN-α (IU) | Yield (%) | Total IFN-β (IU) | Yield (%) |
|---|---|---|---|---|---|---|
| Filtrate | 300 | $5.4 \times 10^5$ | $3.4 \times 10^9$ | 100 | $3.0 \times 10^8$ | 100 |
| Concentrate from Ultrafilter | 3 | $2.1 \times 10^5$ | $3.3 \times 10^9$ | 97 | $2.8 \times 10^8$ | 93 |
| Fraction from Column A | 1.5 | 153 | $2.6 \times 10^9$ $(1.7 \times 10^7)*$ | 77 | $<3 \times 10^6$ | <1 |
| Fraction from Column B | 0.3 | 52 | $<3 \times 10^7$ | <1 | $<2.1 \times 10^8$ $(4.0 \times 10^6)*$ | 70 |

* Specific activity = IU/(total protein)

## EXAMPLE 2

IFN-γ and TNF derived from leucocytes

Production System:

PHA (10 mg/mℓ) was added to a suspension of leucocytes in RPMI1640 medium and the cells were incubated at 37°C for three days to produce IFN-γ and TNF. The volume of culture solution was 300 liters.

Purification Procedure:

The apparatus shown in Figure 1 was used, in which three types of filters were connected: a prefilter of 100 μm pore size (Model U100 AW19.5AC; Brunswick International), a prefilter of 5.0 μm pore size (Model U5A19.5AC; Brunswick International) and a filter of 3.0 μm pore size (Model AB2U030; Pole Inc.). Purification was carried out using an ultrafilter Pellicon® (cassette filter: PTGC) produced by Millipore Inc. Two types of columns (A and B) were employed. Column A was packed with 100 mℓ of silica gel (Microbeads Silica Gel, 500 Å; Fuji-Devison) with monoclonal antibody against IFN-γ fixed thereto, and column B was packed with 100 mℓ of the same silica gel as above with monoclonal antibody against TNF fixed thereto.

A 0.1 M phosphate buffer containing 0.3 M NaCℓ (pH 7.0) was used as the column equilibrating and washing solutions ($A_1$ and $B_1$) each in an amount ten times the volume of the column.

3.5 M KSCN solution (pH 8.0) was used as the column eluent ($A_2$ and $B_2$) each in an amount three times the volume of the column.

The time required for treatment was three hours.

Result of Purification:

IFN-γ and TNF were isolated and purified simultaneously as shown in Table 3.

TABLE 3

| Sample | Volume ($\ell$) | Total Protein ($A_{280\ nm}$) | Total IFN-γ (IU) | Yield (%) | TNF (U) | Yield (%) |
|---|---|---|---|---|---|---|
| Filtrate | 50 | $1.2 \times 10^5$ | $1.5 \times 10^8$ | 100 | $1.4 \times 10^7$ | 100 |
| Concentrate from Ultrafilter | 2 | $0.5 \times 10^5$ | $1.4 \times 10^8$ | 93 | $1.1 \times 10^7$ | 78 |
| Fraction from Column A | 0.3 | 17 | $1.1 \times 10^8$ $(6.5 \times 10^6)*$ | 73 | $<0.4 \times 10^6$ | <3 |
| Fraction from Column B | 0.3 | 24 | $<3 \times 10^6$ | <2 | $<9.0 \times 10^6$ $(3.8 \times 10^5)*$ | 64 |

* Specific activity = IU/(total protein)

EXAMPLE 3

IFN-2α derived from Escherichia coli incorporating recombinant gene

Microorganism:

Escherichia coli C600/plα2 Trp 41 strain (obtained by introducing human IFN-α2 plasmid to E. coli C600 strain, Green Cross Croporation) was used. The volume of the culture solution was 50 liters.

Since IFN-α2 is accumulated inside the microbial cells, the cells must be broken prior to purification (pretreatment). The grown cells were suspended in an extractive buffer solution* (1/5 the volume of culture solution), the suspension was treated in DYNOMILL® to effect attrition and extraction, and the extract (10 liters) was stored for purification.

* 0.25 M Tris + 0.03 M NaCl + 0.05 M EDTA (containing 10 µg/ml phenylmethanesulfonyl fluoride)

Purification Procedure:

The apparatus shown in Figure 2 was used. The concentration step was omitted because the volume to be treated was small. Only one column was used, which was packed with 100 ml of silica gel (Microbeads Silica Gel, 500 Å; Fuji-Devison) with polyclonal antibody against IFN-α fixed thereto. The volume of the column was 500 ml. Five liters of 0.1 M phosphate buffer containing 0.3 M NaCl (pH 7.0) was used as the column equilibrating

and washing solution (A$_1$), and 1.5 liters of 0.1 m citrate buffer (pH 2.0) was used as the column eluent

(A$_2$).

The time required for treatment was 1.5 hours.

Result of Purification:

The result obtained was shown in Table 4.

TABLE 4

| Sample | Volume ($\ell$) | Total Protein (A$_{280 nm}$) | IFN-α2 | | | Degree of Purification |
|---|---|---|---|---|---|---|
| | | | Potency (U) | Yield (%) | Specific Activity (U/total protein) | |
| Eluate | 10 | $4.6 \times 10$ | $6 \times 10^{10}$ | 100 | $1.3 \times 10^5$ | × 1 |
| Eluted Fraction | 1.5 | $2.5 \times 10^3$ | $4.3 \times 10^{10}$ | 72 | $1.3 \times 10^5$ | × 130 |

Escherichia coli K12 strain was used in the succeeding examples      on      sterilization, and its effect was evaluated by the number of colonies measured with a water sampler (Coli-count; Millipore Inc.).

EXAMPLE 4

Polyclonal antibody against IFN-α, obtained from horses immunized with pure IFN-α, was fixed to previously silanized silica gel (Microbeads Silica Gel, 500 Å; Fuji-Devison), 2 grams of this gel was suspended in aqueous solutions of the sugars and sugar alcohols (30 to 90% (w/v)) and of the amino acids (5 to 20% (w/v)) shown in Table 5. To each of the resulting suspensions was added a dilute suspension of Escherichia coli, the mixture was heated at 60°C for 1 to 15 hours at pH 7, and the number of colonies left was measured. The results are summarized in Table 5.

## TABLE 5

### Sterilization of Antibody-Fixed Carrier in the Presence of Sugars, Sugar Alcohols and Amino Acids

| Additive | Concentration (%) | Heating Time (hr) at 60°C | | |
|---|---|---|---|---|
| | | 1 | 3 | 15 |
| Sucrose | 30 | 0 | 0 | 0 |
| | 90 | 2 | 0 | 0 |
| Maltose | 30 | 4 | 0 | 0 |
| | 90 | 3 | 0 | 0 |
| Raffinose | 30 | 3 | 0 | 0 |
| | 90 | 0 | 0 | 0 |
| Sorbitol | 30 | 0 | 0 | 0 |
| | 90 | 2 | 0 | 0 |
| Glycine | 5 | 0 | 0 | 0 |
| | 20 | 4 | 0 | 0 |
| Alanine | 5 | 2 | 0 | 0 |
| | 20 | 4 | 0 | 0 |
| Arginine | 5 | 2 | 0 | 0 |
| | 20 | 4 | 0 | 0 |
| Lysine | 5 | 0 | 0 | 0 |
| | 20 | 3 | 0 | 0 |

Concentration of E. coli before heating: 7811 colony/mℓ Numerals in the table: Concentration of live E. coli after heating (colony/mℓ)

EXAMPLE 5

A dilute suspension of Escherichia coli was added to aqueous solutions (200 mℓ each) of the sugars and sugar alcohols (60% (w/v)) and of amino acids (20% (w/v)), shown in Talbe 6 and the resulting mixture was circulated through a cartridge containing ZETAFFINITY® (Cuno Inc.) with monoclonal antibody against IFN-γ fixed thereto by the periodic acid method, followed by heat treatment at 55 to 70°C for 10 hours at pH 7. No live bacteria (E. coli) was detected after treatment at 60 to 70°C as shown in Table 6.

### TABLE 6

### Sterilization of Antibody-Fixed Carrier by Heating for Ten Hours

| Stabilizer | Concentration (%) | Heating Temperature (°C) | | |
|---|---|---|---|---|
| | | 55 | 60 | 70 |
| Sorbitol | 60 | 1 | 0 | 0 |
| Sucrose | 60 | 0 | 0 | 0 |
| Glycine | 20 | 0 | 0 | 0 |
| Lysine | 20 | 1 | 0 | 0 |

Concentration of E. coli before heating: 4594 colony/mℓ Numerals in the table: Concentration of live E. coli after heating (colony/mℓ)

## EXAMPLE 6

The stability of antibody-fixed carriers against thermal sterilization was examined in this example.

(1) Silica gel with polyclonal antibody against IFN-α fixed thereto by glutaraldehyde method after chemically modifying silica gel with a silan coupling agent (γ-glycidoxypropyltrimethoxysilane) was heated at 60°C for 10 hours in a 60% (w/v) aqueous solution of sorbitol at pH 7. The heat-treated carrier was used for purification of IFN-α, and its recovery rate was compared with the value for an untreated carrier.

The two samples of carriers (heat-treated and untreated) were each charged in a column (10 × 20 mm), and equilibrated with 0.1 M phosphate buffer (pH 7.5). Crude IFN-α was then added and adsorbed on the carrier, unadsorbed proteins and other impurities were removed by washing with the same buffer as above, and the adsorbed IFN-α was eluted with a 0.1 M citrate buffer (pH 2.0). The results are shown in Table 7. As is apparent from Table 7, the carrier retained its antigen-fixing capacity after thermal treatment.

TABLE 7

|  | IFN-α (IU) | Recovery Rate (%) |
|---|---|---|
| Crude IFN-α | $2,200 \times 10^4$ | 100 |
| Heat-Treated Column | $1,760 \times 10^4$ | 80 |
| Untreated Column | $1,720 \times 10^4$ | 78 |

(2) Formylcellulofine (Seikagaku Kogyo) with monoclonal antibody against TNF fixed thereto by the formylation method was heated at 60°C for 10 hours in 60% (w/v) aqueous solution of sucrose at pH 7. The heat-treated carrier was used for purification of TNF, and its recovery rate was compared with the value for an untreated carrier.

The two samples of carriers (heat-treated and untreated) were each charged in a column (10 × 20 mm), and equilibrated with 0.1 M phosphate buffer (pH 7.5). Crude TNF was then added and adsorbed on the carrier, unadsorbed proteins and other impurities were removed by washing with the same buffer as above, and the adsorbed TNF was eluted with a 3.5 M KSCN solution (pH 8.0). The results are shown in Table 8.

TABLE 8

|  | TNF (IU) | Recovery Rate (%) |
|---|---|---|
| Crude TNF | $583 \times 10^4$ | 100 |
| Heat-Treated Column | $460 \times 10^4$ | 79 |
| Untreated Column | $431 \times 10^4$ | 74 |

(3) ZETAFFINITY® cartridge (Cuno Inc.) with monoclonal antibody against IFN-γ fixed thereto by glutaraldehyde method after chemically modifying silica gel with a silane agent ( -glycidoxypropyltrimethoxysilane) was heat-treated by circulating a 20% (w/v) aqueous solution of glycine (60°C, pH 7) over a period of 10 hours. The heat-treated cartridge was used for purification of crude IFN-γ, and its recovery rate was compared with the value for an untreated cartridge.

Crude IFN-γ was adsorbed on each of the two cartridges (heat-treated and untreated), unadsorbed proteins and other impurities were removed by washing with the same buffer as above, and the adsorbed IFN-γ was eluted with a 3.5 M KSCN solution (pH 8.0). The results are shown in Table 9.

### TABLE 9

|  | IFN-γ (IU) | Recovery Rate (%) |
|---|---|---|
| Crude IFN-γ | $3,200 \times 10^4$ | 100 |
| Heat-Treated Column | $2,944 \times 10^4$ | 92 |
| Untreated Column | $3,104 \times 10^4$ | 97 |

### EXAMPLE 7

The stability of protein A-fixed carriers against thermal sterilization was examined in this example.

Formylcellulofine (Seikagaku Kogyo) with Protein A-fixed thereto by the formylation method was heated at 60°C for 10 hours in a 60% (w/v) aqueous solution of sorbitol.

The two samples of carriers (heat-treated and untreated) were each packed in a column (10 × 20 mm), and then the column was charged with a human $I_gG$ solution in 0.01 M phosphate buffer (pH 8.0) (concentration: $A_{280} = 1.08$) to fix the human $I_gG$ on the carrier. After washing the column with the same buffer as above, the adsorbed human $I_gG$ was eluted with a 1 M acetic Acid (pH 2). The results obtained are shown in Table 10. As is apparent from Table 10, the carrier retained its protein A-fixing capacity after thermal treatment.

### TABLE 10

|  | $I_gG$ ($A_{280}$) | Recovery Rate (%) |
|---|---|---|
| Human $I_gG$ Solution | 19.5 | 100 |
| Heat-Treated Column | 11.7 | 60 |
| Untreated Column | 12.3 | 13 |

While the invention has been described in detail and with reference to specific embodiments thereof, it will be apparent to one skilled in the art that various changes and modifications can be made therein without departing from the spirit and scope thereof.

CLAIMS

1.  An automatic process for purifying a physiologically active substance comprising the following steps:

(1)  filtering a sample solution containing said physiologically active substance through at least one filter of a pore size in the range of from 0.22 to 500 µm to give a clarified solution containing said physiologically active substance,

(2)  subjecting the clarified solution thus obtained to ultrafiltration, thereby filtering off impurity substances having a molecular weight lower than that of said physiologically active substance so as to produce a concentrated solution of said physiologically active substance,

(3)  subjecting the concentrated solution containing said physiologically active substance to affinity chromatography, to thereby obtain a purified solution containing said active substance.

2.  An apparatus for automatically purifying a physiologically active substance comprising the following units:

(1)  a tank for storing a solution containing said physiologically active substance to be purified,

(2) a filter with a pore size in the range of from 0.22 to 500 μm for filtering the solution stored in tank (1),

(3) a tank for storing the filtrate obtained from filter (2) and from ultrafilter (4),

(4) an ultrafilter for filtering the solution stored in tank (3) to filter off impurity substances having a molecular weight lower than that of the physiologically active substance being purified, to thereby produce a concentrated solution of said active substance,

(5) a separator through which the concentrated solution obtained above is passed and which contains a carrier with a substance having specific affinity to the physiologically active substance fixed thereto,

(6) a tank to store a washing solution an equilibrating solution, or both for use with separator (5),

(7) a tank for an eluent to elute the phydiologically active substance adsorbed on separator (5),

(8) a tank to store the fractions eluted from separator (5) and containing said physiologically active substance,

(9) pipes to transfer the solutions in tank (1) to filter (2), to tank (3) and from tank (3) to ultrafilter (4). filter (4),

(10)   a pipe to transfer the solution withdrawn from ultrafilter (4) to tank (3),

(11)   pipes to transfer the solutions in tanks (3), (6) and
(7) to separator (5),

(12)   a pipe to transfer the solutions withdrawn from
separator (5) to tank (8) and other units,

(13)   delivery pumps provided in pipes (9), (10) and (11),

(14)   valves provided in pipes (9), (10) and (11),

(15)   liquid level sensors provided in tanks (1) and (3),

(16)   a pressure sensor provided in the pipe between tank
(3) and ultrafilter (4) and in pipe (10), and

(17)   a computer which electrically connected to sensors
(15) and (16) at its input and to valves (14) at its
output.

3.   A carrier with an antibody fixed thereto
for recovering a protein from a cell culture solution,
wherein said carrier is sterilized.

4.   The carrier with a fixed antibody as
defined in Claim 3, wherein said carrier is a member
selected from the group consisting of cellulose, dextran,
porous glass, chitosan, agarose, polyacrylamide and
amino acid copolymers.

5.   The carrier with a fixed antibody as
defined in Claim 3, wherein said carrier is
thermally sterilized at 55 to 70°C for 1 to 15 hours.

6.   The carrier with a fixed antibody as
defined in Claim 3, wherein said thermal sterilization is      conducted in the presence of at least one stabilizer selected from sugars, sugar alcohols and amino acids.

7.   An automatic process for purifying a physiologically active substance comprising the following step:

(1)   filtering a sample solution containing said physiologically active substance through at least one filter of a pore size in the range of from 0.22 to 500 µm to give a clarified solution containing said physiologically active substance, and

(2)   subjecting the clarified solution containing said physiologically active substance to affinity chromatography to thereby obtain a purified solution containing said active substance.

8.   An apparatus for automatically purifying a physiologically active substance comprising the follwoing units:

(1)   a tank for storing a solution containing said physiologically active substance to be purified,

(2)   a filter with a pore size in the range of from 0.22 to 500 µm for filtering the solution stored in tank (1),

(3)   a tank for storing the filtrate obtained from filter (2),

(4)  a separator through which the filtrate obtained above is passed and which contains a carrier with a substance having specific affinity to the physiologically active substance fixed thereto,

(5)  a tank to store a washing solution, an equilibrating solution, or both for use with separator (4),

(6)  a tank for an eluent to elute the physiologically active substance adsorbed on separator (4),

(7)  a tank to store the fractions eluted from separator (4) and containing said physiologically active substance,

(8)  pipes to transfer the solutions in tank (1) to filter (2) to tand (3),

(9)  pipes to transfer the solutions in tanks (3), (5) and (6) to separator (4),

(10) a pipe to transfer the solutions withdrawn from separator (4) to tank (7),

(11) delivery pumps provided in pipes (8), (9) and (10),

(12) valves provided in pipes (8) and (9),

(13) liquid level sensors provided in tanks (1) and (3),

(14) a computer which is electrically connected to sensor (13) at its input and to valve (12) at its output.

9.  The automatic process as claimed in Claim 1, wherein said sample solution is selected from the group consisting of culture solutions of leucocytes, lymphoblasts, hybridomas, E. coli, B. subtilis and yeast.

10. The automatic process as claimed in Claim 7, wherein said sample solution is selected from the group consisting of culture solutions of leucocytes, lymphoblasts, hybridomas, E. coli, B. subtilis and yeast.

11. The automatic process as claimed in Claim 1, wherein said physiologically active substance is selected from the group consisting of interferon, urokinase, prourokinase, kallikrein, lysozyme, trypsin inhibitor, human granulocyte-differentiation-inducing glycoprotein, interleukin, colony-formation-stimulating factor, tissue plasminogen-activating factor, B-cell growth factor, tumor necrosis factor, epidermal growth factor, lymphotoxin, hormone, monoclonal antibody, erythropoietin, antithrombin III, transferrin, plasminogen, plasminogen activator, α-fetoprotein and surface antigen for hepatitis type-B virus.

12. The automatic process as claimed in Claim 7, wherein said physiologically active substance is selected from the group consisting of interferon, urokinase, prourokinase, kallikrein, lysozyme, trypsin inhibitor, human granulocyte-differentiation-inducing glycoprotein, interleukin, colony-formation-stimulating factor, tissue plasminogen-activating factor, B-cell growth factor, tumor necrosis factor, epidermal growth factor, lymphotoxin, hormone, monoclonal antibody, erythropoietin, antithrombin III, transferrin, plasminogen, plasminogen activator, α-fetoprotein and surface antigen for hepatitis type-B virus.

13. The automatic process as claimed in Claim 1, wherein filters of 3 μm, 0.45 μm and 0.22 μm in pore size are used sequentially.

14. The automatic process as claimed in Claim 7, wherein filters of 3 μm, 0.45 μm and 0.22 μm in pore size are used sequentially.

15. The automatic process as claimed in Claim 1, wherein filters of 100 μm, 5 μm, 3 μm, 0.45 μm and 0.22 μm in pore size are used sequentially.

16. The automatic process as claimed in Claim 7, wherein filters of 100 μm, 5 μm, 3 μm, 0.45 μm and 0.22 μm in pore size are used sequentially.

17. The automatic process as claimed in Claim 1, wherein filters of 0.45 μm and 0.22 μm in pore sizes are used sequentially.

18. The automatic process as claimed in Claim 7, wherein filters of 0.45 μm and 0.22 μm in pore size are used sequentially.

Fig. 1

Fig. 2

culture tank or
crude bulk pool tank
1

clean steam
clean air
sterile water

heat exchanger

cooling coil

5

ultra-filter

Pellicon® (fraction molecular weight: $10^4$)

column A

column B

eluate fraction

washing solution

eluent

drain

4 filter
$d = 0.22$
Prostac

eluate fraction

$P$: pump for delivering washing solution
$P$: pressure sensor
$L$: liquid surface sensor
concentration step which can be omitted
$\bowtie$: valve
$\square$: filter
$\boxtimes$: ultrafilter
$d$: pore size (µm)

Fig. 3: Flowchart for Computor C

**0 249 932**

start

**Filtration Step**

- actuate pump 2
- check if liquid remains in Tank 1 → yes → check if liquid level in filter 4 is above predetermined level → no → check if liquid amount in Tank 5 is above predetermined level → no
  - yes → temporarily stop pump 2
- no → stop pump 2

**Concentration Step**

- open valve on the side of filter 6
- actuate pump on the side of filter 6
- check if liquid level in Tank 5 is above predetermined level → yes → check if liquid pressure on the side of tank 5 is above predetermined level → no
  - yes → temporarily stop pump on the side of filter 6
- no → stop pump on the side of filter 6
- close valve on the side of filter 6

**Introduction Step (To Column)**

- open valve on the side of pump 7
- actuate pump 7
- check if liquid remains in Tank 5 → Yes → check if liquid pressure on the side of pump 7 is above predetermined level → no
  - yes → temporarily stop pump 7
- no → stop pump 7

- washing step
- eluting step

end

Detection by Sensor

Motion of Valve or Pump